(19) 

**Europäisches Patentamt**
**European Patent Office**
**Office européen des brevets**

(11) **EP 4 531 430 A1**

(12) **EUROPEAN PATENT APPLICATION**

(43) Date of publication:
**02.04.2025 Bulletin 2025/14**

(21) Application number: **24203379.3**

(22) Date of filing: **27.09.2024**

(51) International Patent Classification (IPC):
**H04R 1/10** *(2006.01)* **H04R 1/28** *(2006.01)*
**A61F 11/08** *(2006.01)*

(52) Cooperative Patent Classification (CPC):
**H04R 1/1016; A61F 11/08; H04R 1/1083;**
**H04R 1/2803; H04R 2420/07; H04R 2460/11**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **28.09.2023 US 202363586161 P**

(71) Applicant: **Red Tail Hawk Corporation**
**Philadelphia, PA 19107 (US)**

(72) Inventor: **Parkins, John W**
**Philadelphia, 19107 (US)**

(74) Representative: **Patent Boutique LLP**
**10A Printing House Yard**
**Hackney Road**
**London E2 7PR (GB)**

(54) **WIRELESS BATTERY-FREE MAGNETIC INDUCTION EARPLUG WITH EXPANSION CAVITY**

(57) A battery-free magnetic induction earplug includes a housing defining a main cavity, a sound output channel connected to the main cavity, and an expansion cavity. The battery-free magnetic induction earplug can also include a receiver in the main cavity, and can include a speaker in the main cavity adjacent the receiver, the receiver including a bobbin and a coil of wire wound upon the bobbin, the speaker acoustically coupled to the sound output channel, the speaker having a diaphragm and a volume upstream from the diaphragm, and the speaker having a vent from the volume to the expansion cavity.

Fig. 3

EP 4 531 430 A1

## Description

### REFERENCE TO RELATED APPLICATIONS

This application claims one or more inventions disclosed in Provisional Application Number 63/586,161, filed September 28, 2023, titled " Wireless Battery-Free Magnetic Induction Earplug With Expansion Cavity". The benefit under 35 USC §119(e) of the United States provisional application is hereby claimed, and the aforementioned application is hereby incorporated herein by reference.

### BACKGROUND OF THE INVENTION

### FIELD OF THE INVENTION

The invention pertains to the field of communications earplugs. More particularly, the invention pertains to a wireless battery-free earplug for use with a magnetic induction communications system.

### DESCRIPTION OF RELATED ART

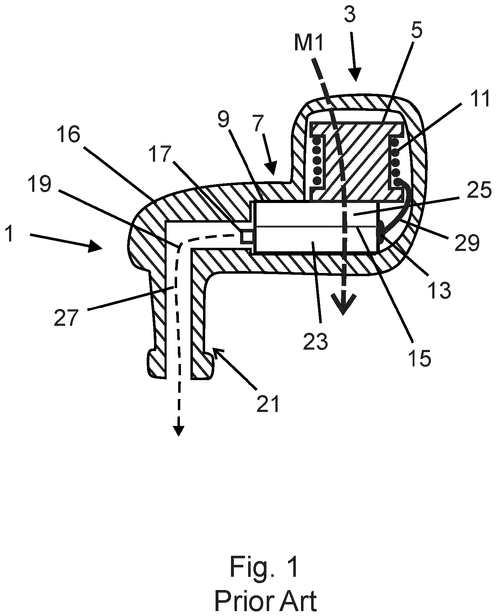

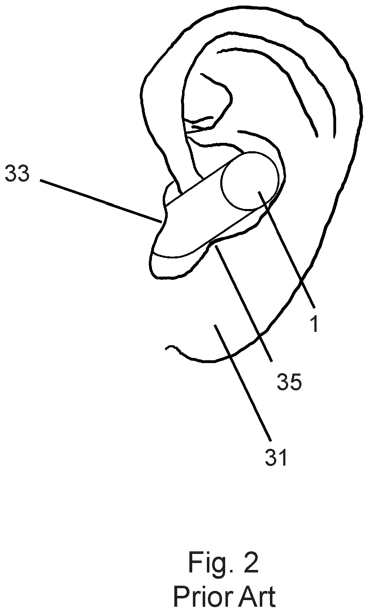

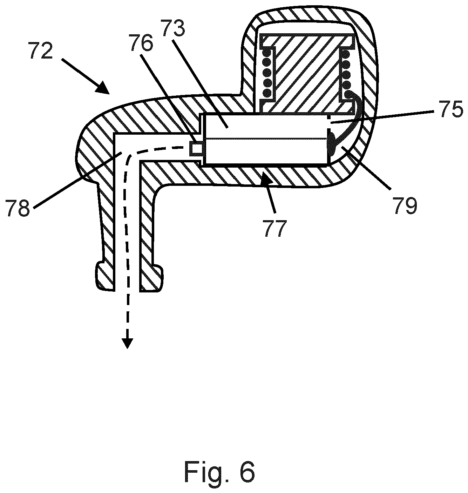

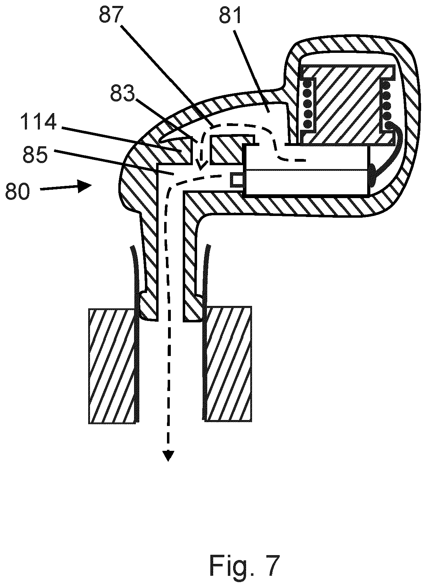

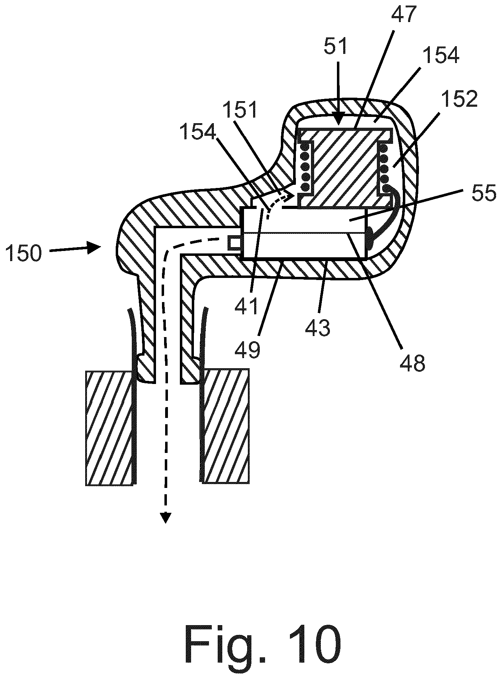

Wireless battery-free magnetic induction (BMI) earplugs have been described in patents such as US10,448,143B, US10,357,403B2, US9,083,388, and USD657,499S. As seen in Fig. 1 and Fig. 2 of US10,357,403B2, a transmitter coil 1 is used to communicate with a receiver coil 21 using magnetic fields. Fig. 10 of US10,357,403B2 shows a receiver coil 62 wrapped around a bobbin 63 of magnetic material. The transmitter coil 1 of Fig. 1 is used to generate audio frequency magnetic fields that produce sound in an ear canal using a wireless earplug 64 shown in Fig. 7. The wireless earplug 64 has an eartip 60, seen in Fig. 6 that protects the user from undesirable ambient acoustic noise. There is no battery in the earplug. Thus, hearing protection with wireless communications can be provided using an earplug without needing a battery in the earplug.

The term "magnetic material" includes all low-reluctance material and the material need not be magnetized to create a permanent magnet, as is known in the art. As an example, some stainless steel materials have low reluctance and are considered to be magnetic materials even if they are not magnetized.

BMI earplugs can have various geometries that result from the relative position and orientation of the receiver coil and speaker. In Fig. 10 of US10,357,403B2, the relative position and orientation of the receiver coil 62 and the speaker 61 results in an "L-shape" geometry earplug (when excluding the eartip 60 and eartip adapter 65). The "L-shape" geometry corresponds with offset center axes of the receiver and the speaker. Figures 3-5 in US10,357,403B2 show a BMI earplug with an arrangement of a receiver coil 21 and a speaker 23 that does not form an "L-shape" geometry. Instead, the receiver coil 21 wraps around the speaker 23 such that the center axis of each is aligned. This arrangement forms more of an ellipsoid geometry (excluding the eartip 35 and the eartip adapter 44). Figs. 1 and 2 of US9,525,930 show yet another geometry that can be used as a BMI earplug. In Figs. 1 and 2 of US9,525,930, the receiver coil 24 is positioned directly adjacent a cylindrical speaker 16, with center axes of each aligned, forming a cylindrical earplug (excluding the eartip 10 and the eartip adapter 36).

Fig. 1 shows a cross section of a conventional BMI earplug 1. A magnetic field transmitter (not shown) such as a coil generates a magnetic field M1. A receiver 3 having a bobbin 5 made of magnetic material is located above a speaker 7 having a speaker case 9 at least partially made of magnetic material. The bobbin 5 of the receiver 3 is mounted in sufficiently close proximity to the case 9 of the speaker 7 and oriented such that magnetic flux lines passing through the speaker 7 also pass through a coil 11 of the receiver, the coil wrapping or spiraling around the bobbin 5. This orientation of the receiver 3 and the speaker 7 facilitates passage of the magnetic field lines through the receiver coil 11 due to the low reluctance path provided by the magnetic materials of the receiver bobbin 5 and the speaker 7. The geometry and orientation of the receiver coil 11 with the speaker 7 in this BMI earplug 1 creates a particularly efficient BMI earplug. The receiver 3, the speaker 7, and the case 9, and/or other components of the BMI earplug 1 are housed in an earplug housing 16.

The receiver coil 11 converts the magnetic field M1, comprised of signals in the audio spectrum, to a voltage, which is input to the speaker 7 at a speaker input 13 using a receiver wire 29. A speaker typically requires more than one speaker input, but only one speaker input 13 is shown in Fig. 1 for clarity. Additional speaker components located within the speaker case 9, such as the speaker suspension and coil are also not shown for clarity. The speaker 7 has a speaker diaphragm 15 that moves when a voltage is present at the speaker input 13. The speaker 7 generates sound based on the magnetic field signal, providing communication in the form of speech, music, or other audio spectrum content. The audio spectrum is typically defined in the art as 20 Hz to 20 kHz. The sound generated by the speaker 7 exits the speaker 7 through a sound port 17 and travels to an ear canal (not shown) through a sound channel 19. An eartip (not shown), such as those described in US10,357,403B2, is attached and secured to an eartip adapter 21 of the BMI earplug 1. The eartip (not shown) seals against the ear canal (not shown), preventing ambient noise from entering the ear canal while desired communications are passed through the sound channel 19 and a hole (not shown) through the eartip, as described in US10,357,403B2. Thus, communications can be provided to a user of an earplug device incorporating the BMI earplug 1 to protect human hearing without earplug communication wires or earplug batteries. Earplug batteries and communication wires are generally considered to be disadvantageous, as is known in the art.

When a speaker diaphragm 15 moves toward the sound port 17 (toward the forward or downstream direction), the speaker 7 generates positive pressure in a port side volume (or front volume or downstream volume) 23 of the speaker diaphragm 15, and generates a negative pressure on an opposite side of the diaphragm (behind the diaphragm or on the upstream side of the speaker diaphragm 15). To prevent the positive and negative pressures from equalizing and diminishing the sound output, an enclosure with a back volume (or upstream volume) 25, is often used to contain the pressure behind the speaker diaphragm 15. This design is often used in home stereo applications, in which the speaker takes the form of a box with the front (port/downstream side) of the speaker diaphragm communicating with the room and listener while the back (upstream side) of the speaker diaphragm communicates with a speaker back volume enclosed by the speaker box. In this way, pressure generated at the back of the diaphragm does not reduce the desirable musical content generated by the front of the diaphragm that ultimately reaches the listener.

Miniature speakers may incorporate a speaker front volume 23, as shown in Fig. 1, to protect the speaker diaphragm 15 and/or channel the sound output to a desired location. In the embodiment of Fig. 1, the speaker front volume 23 and the speaker back volume 25 are surrounded by a speaker case 9 that defines the overall geometry of the outer surface of the speaker. The sound port 17 sealed to the speaker front volume 23 is used to direct the speaker acoustical output from the speaker front volume 23 into a sound channel 27.

When a speaker back volume 25 is used, it imposes an impedance to movement of the speaker diaphragm 15 unless the acoustical stiffness of the speaker back volume 25 is sufficiently small compared to the stiffness of the speaker diaphragm 15, as is known in the art. However, the back volume 25 of small speakers typically causes a speaker to be less efficient compared to a speaker with a large speaker back volume.

Although a BMI earplug 1 requires no battery to provide communications, a battery located outside of the BMI earplug 1 may be needed to provide power to an amplifier for the audio signal used to drive the transmitter coil located outside the BMI earplug 1. The higher the efficiency of the BMI earplug 1 in converting the receiver coil 11 voltage to sound, the less energy is needed to drive the amplifier external to the BMI earplug 1. A BMI earplug 1 efficiency improvement of only 3 dB will result in approximately half the power needed. Thus, if a battery is used to power an amplifier for the transmitter signal, then the battery duration would last about twice as long with a 3 dB sensitivity improvement. Accordingly, improving the efficiency of BMI earplugs is highly desirable in applications using a battery outside the earplug to amplify the audio signal delivered to the transmitter coil.

Transmitter coils typically have an electrical impedance that can be modeled as a resistor in series with an inductor, when capacitance is neglected, which is a reasonable assumption at lower frequencies, as is known in the art. The impedance of the transmitter coil in this case is

$$Ztc = 2\pi fL + R \qquad (1),$$

where L is the effective inductance, R is the effective resistance, and f is the frequency. This impedance becomes approximately constant at low frequencies and approximately equal to R. At frequencies significantly above the break frequency (fb = $L/2\pi R$), however, the impedance generally increases linearly as a function of frequency, as indicated in Eq. 1 and as known in the art. However, the received voltage generated by the receiver coil, Vout in Fig. 2 of US10,357,403B2, and at speaker input 13 in Fig. 1 (Prior Art), is generally proportional to the transmitter coil voltage, Vin in Fig. 2 of US10,357,403B2, substantially above fb. Below fb, the system efficiency in generating sound decreases, due to resistive losses in the transmitter coil and the lower impedance compared to high frequencies. Thus, the energy used to drive the transmitter coil for the same acoustical output in a BMI earplug system is often dominated by lower frequencies, and improving the sensitivity of the earplug at these frequencies can significantly improve the efficiency of the BMI earplug system. This function is important if an external battery is used to amplify the audio signal delivered to the transmitter coil in Fig. 2 of US10,357,403B2.

Comfort is of crucial importance to user acceptance of earplugs. Yuan, et al., in a paper titled "Measurement of Pressure Discomfort Threshold in Auricular Concha for In-Ear Wearables Design" in Applied Ergonomics (2023) (10.1016/j.apergo.2023.104078) asserts "The present study identified low pressure sensitivity in the concha, high pressure sensitivity in the limbic region and the highest pressure sensitivity in the tragus." From Table 2 in the paper, it can be seen that the tragus is the most sensitive region in the ear for both men and women. In women, the antitragus is the second most sensitive region, while in men it is the third most sensitive. Men are more likely to have larger ears than women so men are less likely to experience discomfort due to contact pressure for a given earplug design. For both men and women, though, to avoid discomfort and achieve broad user acceptance, an earplug must minimize pressure on the tragus and antitragus region. Ideally, for maximum comfort, an earplug should minimize or avoid contact with these regions.

All other parameters being equal, larger speakers are generally more efficient compared to smaller speakers due to the larger size of the speaker diaphragm, as is known in the art. Thus, a larger speaker can be used to reduce power requirements of a communications earplug design. Using larger speakers increases the physical size of an earplug, though, which can cause discomfort. Further, a given speaker geometry is fixed and the designer has limited options regarding the overall earplug shape, which causes difficulty or impossibility creating a comfortable earplug using a larger speaker.

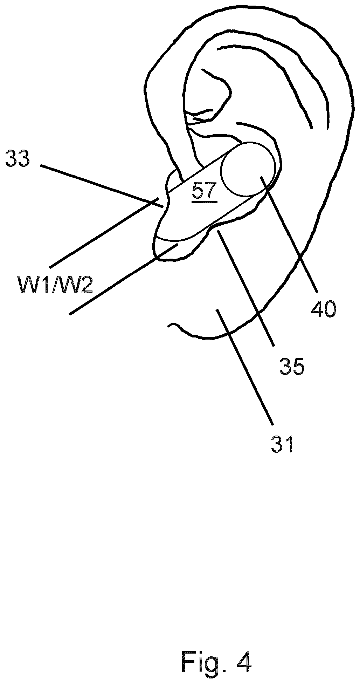

Fig. 2 shows the BMI earplug 1 worn in a small human ear 31. Figure 2 shows the location of the tragus 33 and antitragus 35 and how the BMI earplug 1 can fit in the small human ear 31, the fit being more difficult than for a large ear. An eartip (not shown), similar to the eartip shown in Fig. 4 of US10,357,403B2, would be used to seal the earplug in the ear canal (not shown). The BMI earplug 1 fits comfortably in the artificial ear 31 and makes almost no contact with the tragus 33 and antitragus 35. A relatively small amount of space is available for a larger speaker that could improve sensitivity, but a larger speaker would also decrease comfort for the user.

## SUMMARY OF THE INVENTION

A BMI earplug is provided that improves sensitivity while reducing, minimizing, or avoiding an increase in discomfort. An improved wireless communications earplug is provided for use with a magnetic field transmitter, the improved wireless communications earplug having improved efficiency facilitated by an expansion cavity. In an embodiment, high comfort is facilitated by strategically locating an expansion cavity above the speaker and adjacent the receiver coil with contoured geometry to generally fit between the tragus and antitragus when worn in a human ear. The geometry of the expansion cavity in one embodiment is particularly size-efficient when used with the geometry of a BMI earplug. Namely, the geometry and orientation of the expansion cavity is blended with the geometry and orientation of a high efficiency BMI earplug to yield exceptional sensitivity.

In an embodiment, a battery-free magnetic induction earplug includes a housing defining a main cavity, a sound output channel connected to the main cavity, and an expansion cavity. The battery-free magnetic induction earplug also includes a receiver in the main cavity, the receiver including a bobbin and a coil of wire wound upon the bobbin. A speaker is in the main cavity adjacent the receiver, the speaker acoustically coupled to the sound output channel, the speaker having a diaphragm and a volume upstream from the diaphragm, the speaker having a vent from the volume to the expansion cavity.

In an embodiment, a battery-free magnetic induction earplug includes a housing defining a main cavity, a sound output channel connected to the main cavity, and an expansion cavity connected to the main cavity. The housing includes a first wall between the expansion cavity and the sound output channel.

## BRIEF DESCRIPTION OF THE DRAWINGS

Fig. 1 shows a cross section of a conventional BMI earplug.

Fig. 2 shows the BMI earplug of Fig. 1 in a human ear.

Fig. 3 shows a cross-section of a BMI earplug, according to an embodiment of the invention.

Fig. 4 shows a perspective view of the BMI earplug of Fig. 3 in a human ear.

Fig. 5 shows a cross-section of a BMI earplug, according to an embodiment incorporating a sound port acoustically coupling a coil cavity with an expansion cavity and a speaker back cavity.

Fig. 6 shows a cross-section of a BMI earplug, according to an embodiment in which a speaker incorporates a vent near a back of the speaker.

Fig. 7 shows a cross-section of a BMI earplug, according to an embodiment incorporating a sound port that acoustically couples an expansion cavity with a sound channel.

Fig. 8 shows a cross-section of a BMI earplug, according to an embodiment incorporating a front chamber.

Fig. 9 shows a cross-section of a BMI earplug, according to an embodiment reducing internal structure of an earplug housing.

Fig. 10 shows a cross-section of a BMI earplug, according to an embodiment incorporating a vent from a speaker to a coil cavity.

## DETAILED DESCRIPTION OF THE INVENTION

The terminology used herein is for the purpose of describing particular example embodiments only and is not intended to be limiting. As used herein, the singular forms "a", "an", and "the" may be intended to include the plural forms as well, unless the context clearly indicates otherwise. The terms "comprises," "comprising," "including," and "having," are inclusive and therefore specify the presence of stated features, integers, steps, operations, elements, and/or components, but do not preclude the presence or addition of one or more other features, integers, steps, operations, elements, components, and/or groups thereof. The method steps, processes, and operations described herein are not to be construed as necessarily requiring their performance in the particular order discussed or illustrated, unless specifically identified as an order of performance. It is also to be understood that additional or alternative steps may be employed.

When an element or layer is referred to as being "on", "engaged to" "connected to" or "coupled to" another element or layer, it may be directly on, engaged, connected or coupled to the other element or layer, or intervening elements or layers may be present. In contrast, when an element is referred to as being "directly on," "directly engaged to", "directly connected to" or "directly coupled to" another element or layer, there may be no intervening elements or layers present. Other words used to describe the relationship between elements should be interpreted in a like fashion (e.g., "between" versus "directly between," "adjacent" versus "directly adjacent," etc.). As used herein, the term "and/or" includes any and all combinations of one or more of the associated listed items.

Spatially relative terms, such as "inner," "outer," "beneath", "below", "lower", "above", "upper" and the like, may be used herein for ease of description to describe one element or feature's relationship to another element(s) or feature(s) as illustrated in the Figures. Spatially relative terms may be intended to encompass different orientations of the device in use or operation in addition to the orientation depicted in the Figures. For example, if the device in the Figures is turned over, elements described as "below" or "beneath" other elements or features would then be oriented "above" the other elements or features. Thus, the example term "below" can encompass both an orientation of above and below. The device may be otherwise oriented (rotated 90 degrees or at other orientations) and the spatially relative descriptors used herein interpreted accordingly.

Notwithstanding that the numerical ranges and parameters setting forth the broad scope of embodiments are approximations, the numerical values set forth in the specific examples are reported as precisely as possible. Any numerical value, however, inherently contains an error tolerance necessarily resulting from the standard deviation found in its testing measurements. Moreover, all ranges disclosed herein are to be understood to encompass any and all sub-ranges subsumed therein. For example, a range of "less than 10" can include any and all sub-ranges between (and including) the minimum value of zero and the maximum value of 10, that is, any and all sub-ranges having a minimum value of equal to or greater than zero and a maximum value of equal to or less than 10, e.g., 1 to 5. In certain cases, the numerical values as stated for the parameter can take on negative values. In this case, the example value of range stated as "less than 10" can assume negative values, e.g. - 1, -2, -3, -10, -20, -30, etc.

The terms "approximately" and "about", when qualifying a quantity, shall mean the quantity with a tolerance plus or minus 10 percent of the quantity, unless specified otherwise.

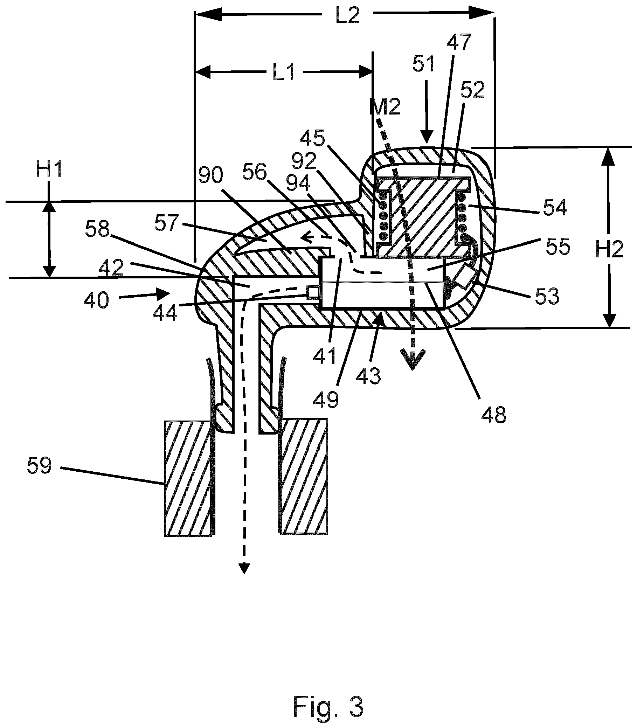

Fig. 3 shows a cross-section of a BMI earplug 40, which is improved over conventional devices, such as the one shown in Figs. 1 and 2. An earplug housing 58 includes or defines therein a main cavity 52 and a sound output channel 42 extending from the main cavity 52. The main cavity 52 houses a receiver 51, which includes a receiver coil 45 wrapped around a receiver bobbin 47, leaving a coil cavity 54 open between the receiver coil 45 and the earplug housing 58. The receiver coil 45 is made of magnetic material. The earplug housing 58 also houses a speaker 43 in the main cavity 52 adjacent the receiver 51. The speaker 43 has a speaker case 49 at least partially constructed of magnetic material metal to direct a magnetic field M2 through the receiver coil 45. A central axis or axis of rotation of the coil 45 is generally perpendicular to a surface of the speaker case 49 forming a generally right angle geometry wherein the axis of the receiver coil 45 and surface of the speaker case 49. The receiver bobbin 47 is mounted in sufficiently close proximity to the speaker case 49 and oriented such that flux lines from the magnetic field M2 passing through the speaker 43 also pass through the receiver coil 45. The orientation of the receiver 51 with respect to the speaker 43 facilitates channeling of the magnetic field M2 through the receiver coil 45 by providing a low reluctance path. Namely, both the receiver bobbin 47 and the speaker 43 are at least partially constructed of magnetic material and are located to encourage a magnetic field path through the receiver coil 45, which creates a particularly sensitive BMI earplug geometry/orientation.

The receiver coil 45 inputs an electrical signal to the speaker 43 through an equalization circuit 53 such that voltage generated by the receiver coil 45 passing through the equalization circuit 53 drives the speaker 43. Alternatively, the equalization circuit 53 can be omitted and the receiver coil 45 can input an electrical signal directly to the speaker 43.

The earplug housing 58 defines an expansion cavity 57 adjacent both the speaker 43 and the receiver 51, separated from the speaker 43 by a first wall 90 and separated from the receiver 51 by a second wall 92. A length L1 of a portion of the earplug housing 58, including walls of the earplug housing 58, immediately bounding the expansion cavity 57 is limited within a length L2 of the earplug housing 58 where the earplug housing 58 defines the main cavity 52 and the sound output channel 42. A height H1 of a portion of the earplug housing 58, including the walls of the earplug housing 58, immediately bounding the expansion cavity 57 is limited within a length H2 of the earplug housing 58 where the earplug housing 58 defines the main cavity 52. A width W1 (see Fig. 4) of a portion of the earplug housing 58, including walls of the earplug housing, immediately bounding the expansion cavity 57 is limited within a width W2 of the earplug housing 58 where the earplug housing 58 defines the main cavity 52. Further, when the BMI earplug 40 is worn in a human ear 31 (see Fig 4), the exterior portion of the earplug housing 58 that defines the expansion cavity 57 faces outward from the human ear 31 and avoids increasing contact between the earplug housing 58 and the human ear 31.

The expansion cavity 57 acoustically connects with the speaker back cavity 55 through a first opening 94 in the first wall 90. The first opening 94 is aligned with a vent 41 through the speaker case 49. The vent 41 is an opening through the speaker case 49 between the sound output channel 42 and the coil cavity 54. In particular, the vent 41 opens through a surface of the speaker case 49 directly adjacent a surface of the speaker case 49 through which sound can exit a sound port 44 into the sound output channel 42. Sound can travel along a sound path 56 from the speaker back cavity 55 through the vent 41 to the expansion cavity 57. The vent 41 faces, and a general direction of sound travel through the vent 41 is, perpendicular to the direction of sound travel through the sound port 44. In this way, at lower frequencies, the effective volume of the speaker back cavity 55 is increased and the resisting acoustical impedance on a speaker diaphragm 48 is reduced. At high enough frequencies, the vent 41 acts as an acoustical mass and facilitates an increase in acoustical impedance and a diminishing of the effect of the expansion cavity. However, because the efficiency of a BMI earplug system with transmitter is lower at lower frequencies and can dominate the power used by a battery for amplifying the transmitter audio signals, improving the efficiency at low frequencies is particularly beneficial. It does not require a large volume of expansion cavity to reduce the resisting impedance on the speaker diaphragm 48 because the back cavity of a small speaker is typically relatively small. The geometry and orientation of the expansion cavity 57 is blended with the geometry and orientation of the receiver 51 and the speaker 43 to yield exceptional comfort and sensitivity.

An eartip 59 is attached to the earplug 40 by now-known or future-developed means. The eartip 59 can be made of foam, rubber, or other now-known or future-developed materials. The earplug 40 can be embedded in a custom mold.

Still referring to Fig. 3, the equalization circuit 53 is connected to the speaker 43 and the receiver coil 45 to tailor the frequency response of the earplug 40. The use of an equalization circuit to modify earplug response is described in US10,357,403B2. In one embodiment of the invention, the equalization circuit 53 can comprise a single capacitor electrically connected across speaker terminals with the coil input to the speaker terminals. This configuration can be used to boost high frequencies. A capacitor equalization circuit is described in Fig. 3 of US 10,3 57,403B2.

Fig. 4 shows the BMI earplug 40 worn in the small, human ear 31 and how the BMI earplug 40 fits in the ear 31. The expansion cavity 57 is generally located between the tragus 33 and the antitragus 35 in the ear 31 to avoid contact with the tragus 33 and the antitragus 35. Accordingly, the BMI earplug 1 of Figs. 1 and 2 can be modified to add an expansion cavity, which can improve the sensitivity without significantly affecting comfort. Contouring of the earplug housing 58 around the expansion cavity 57 prevents or reduces edges on the external surface of the earplug housing 58 that can protrude into the tragus and antitragus and other areas of the ear. The geometry of the expansion chamber 57 blends with the specific orientation/geometry of the speaker 43 and the receiver 51 to create a comfortable earplug 40. In Fig. 3 and Fig. 4, the height of the earplug in the region of the expansion cavity 57 is most pronounced in the area adjacent the receiver 51, and the shortest portion of the earplug 40 is toward the front (downstream side of the diaphragm 48). The geometry of the expansion cavity 57 is particularly size-efficient when used with the geometry of the receiver 51 and speaker 43 orientation as the expansion cavity 57 fills a region that the tragus 33 and the antitragus 35 may not contact, and results in minimal interference with the tragus 33 and antitragus 35. The region of the earplug 40 where the receiver 51 and the speaker 43 meet is typically not in contact with the ear 31 when the earplug 40 is worn correctly. This combined geometry of the speaker 43, the receiver 51, and the expansion cavity 57 creates an especially comfortable earplug shape.

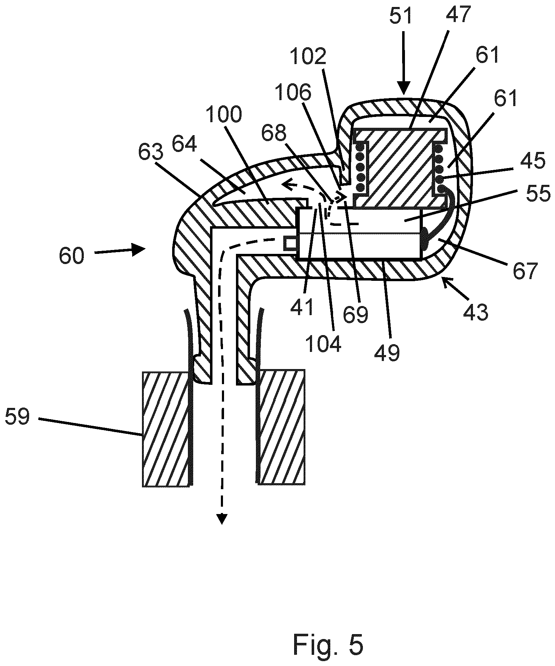

Fig. 5 shows a cross-section of a BMI earplug 60 including an earplug housing 63 that houses components of the BMI earplug 60, such as the receiver 51 and the speaker 43. The earplug housing 63 defines an expansion cavity 64 adjacent both the speaker 43 and the receiver 51, separated from the speaker 43 by a first wall 100 and separated from the receiver 51 by a second wall 102. The expansion cavity 64 acoustically connects with the back cavity 55 of the speaker 43 through a first opening 104 in the first wall 100. The first opening 104 is aligned with the vent 41 through the speaker case 49. The expansion cavity 64 also connects acoustically with a coil cavity 61 around the receiver coil 45 through a second opening 106 in the second wall 102. The first opening 104 and the second opening 106 can be joined or merged, as depicted in Fig. 5, which can facilitate a more unrestricted sound travel and easier manufacturing.

Together, the vent 41, the first opening 104, and the second opening 106 form a sound port 69, which acoustically connects the speaker back cavity 55 and expansion cavity 57 with the coil cavity 61 via a sound path 68. housing A speaker residual cavity 67 between the speaker 43 and the earplug housing 63 is also acoustically coupled to the coil cavity 61. The combined volume of the coil cavity 61 and the speaker residual cavity 67 is relatively small in the embodiment but further increases the effective volume of the expansion cavity 64 and further improves the sensitivity of the BMI earplug 60. The coil cavity 61 can also be used as an expansion cavity in place of the expansion cavity 64.

Fig. 6 shows a cross-section of a BMI earplug 72 including a speaker 77 with a vent 75 opening from a speaker back cavity 73 to a speaker residual cavity 79. The vent 75 opens through a surface of the speaker 77 opposite a surface of the speaker 77 through which sound can exit a sound port 76 into a sound output channel 78. The vent 75 faces, and a general direction of sound travel through the vent 75 is, opposite the direction of sound travel through the sound port 76. In this way, the speaker residual cavity 79 functions as an expansion cavity.

In some embodiments, the expansion cavity is filled with an acoustic absorption material such as foam. The use of acoustic absorption material can absorb acoustical resonances and increase the effective volume of the expansion cavity.

Fig. 7 shows a cross-section of a BMI earplug 80 including a sound port 83 connecting an expansion cavity 81 with a sound output channel 85 through a wall 114 separating the expansion cavity 81 from the sound output channel 85. The sound port 83 facilitates acoustical communication between the expansion cavity 81 and the sound output channel 85. The sound port 83 is sized, having effective length and effective diameter, to act as an acoustical mass. This acoustical mass resonates with the expansion cavity 81 to boost the frequency response at the resonance frequency. Boosting the frequency response at the resonance frequency can improve the sensitivity of the earplug 80 at the resonance frequency but at the expense of diminished sensitivity at frequencies sufficiently below the resonance frequency.

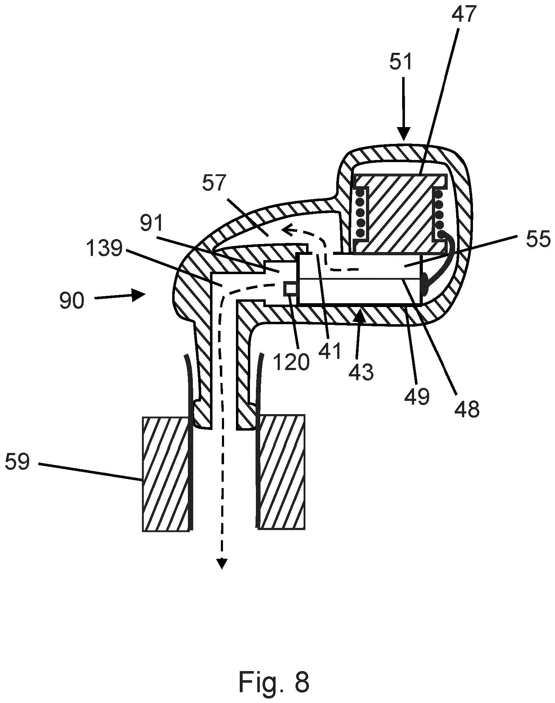

Fig. 8 shows a BMI earplug 90 that includes a speaker output chamber 91 directly adjacent and/or housing a sound port 120 from the speaker 43. The speaker output chamber 91 leads directly from the sound port 120 to a sound output channel 139. The output chamber 91 has a width or diameter (or other radial dimension) larger than that of the sound output channel 139. A volume of the speaker output chamber 91 acts primarily as an acoustical compliance, as opposed to an acoustical mass or acoustical resistance. The speaker output chamber 91 is intended to boost the high frequency response of the BMI earplug 90. This speaker output chamber 91 creates a resonance due to the effective acoustical compliance of the chamber 91 with the effective acoustical mass of the speaker 43 and the sound port 120 to increase the response at high frequencies. The port 83 shown in figure 7 can also be used in combination with this speaker output chamber 91 to achieve both low-frequency boosting and high-frequency boosting.

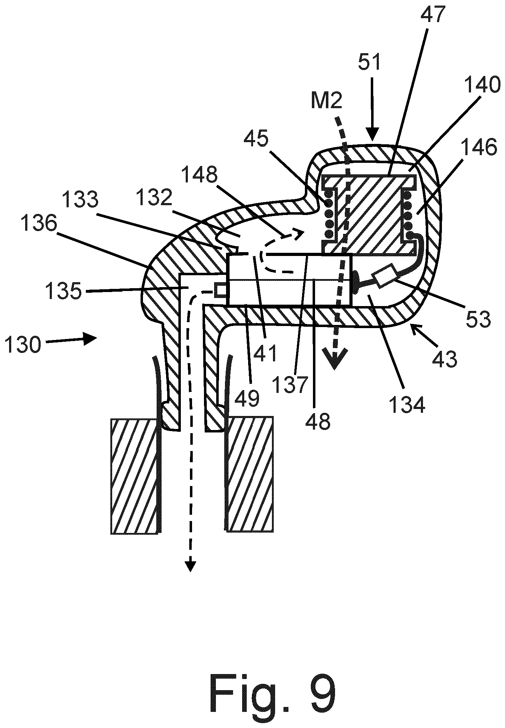

Fig. 9 shows a BMI earplug 130 with an expansion cavity 132 that is an extension of a main cavity 140 with little or no delineation from the main cavity 140. In other words, the expansion cavity 132 has a single relatively large opening extending a majority or all of a length L3 of the expansion cavity 132 and extending a majority or all of a height H3 of the expansion cavity 132. A first wall 133 of an earplug housing 136 separates the expansion cavity 132 from a sound output channel 135. The first wall 133 can extend a relatively short or nominal distance between the expansion cavity 132 and the main cavity 140 or can terminate without extending between the expansion cavity 132 and the main cavity 140. A side 137 of the speaker case 49 partially defines the boundary between the expansion cavity 132 and the main cavity 140, and the vent 41 extends across a minority of the side 137.

Consistent with the expansion cavity 132 having a single relatively large opening extending a majority or all of the height H3 of the expansion cavity 132, the expansion cavity 132 is directly open to a coil cavity 146, which is connected to a speaker cavity 134. A sound path 148 exists through the vent 41 and the expansion cavity 132 to the coil cavity 146. This design, with less internal walls and/or complexity of the earplug housing 136 can be easier to manufacture than BMI earplugs with additional internal walls, due to, e.g., plastic injection molding processes and the way mold pieces slide.

Fig. 10 shows a BMI earplug 150 with an expansion sound channel 151 extending from the speaker 43 to a coil cavity 152. A sound path 154 extends from the vent 41 of the speaker 43 through the expansion sound channel 151 to the coil cavity 152. The coil cavity 152 in combination with the expansion sound channel 151 acts as an expansion cavity in this embodiment. While the invention has been described in terms of particular variations and illustrative figures, those of ordinary skill in the art will recognize that the invention is not limited to the variations or figures described. In addition, where methods and steps described above indicate certain events occurring in certain order, those of ordinary skill in the art will recognize that the ordering of certain steps may be modified and that such modifications are in accordance with the variations of the invention. Additionally, certain of the steps may be performed concurrently in a parallel process when possible, as well as performed sequentially as described above. Therefore, to the extent there are variations of the invention that are within the spirit of the disclosure or equivalent to the inventions found in the claims, it is the intent that this patent will cover those variations as well.

To the extent that the claims recite the phrase "at least one of' in reference to a plurality of elements, this phrase is intended to mean at least one or more of the listed elements and is not limited to at least one of each element. For example, "at least one of an element A, element B, and element C," is intended to indicate element A alone, or element B alone, or element C alone, or any combination thereof. "At least one of element A, element B, and element C" is not intended to be limited to at least one of an element A, at least one of an element B, and at least one of an element C.

This detailed description uses examples to disclose the invention, including the best mode, and to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal language of the claims.

The corresponding structures, materials, acts, and equivalents of all means or step plus function elements in the claims below, if any, are intended to include any structure, material, or act for performing the function in combination with other claimed elements as specifically claimed. The description set forth herein has been presented for purposes of illustration and description but is not intended to be exhaustive or limited to the form disclosed. Many modifications and variations will be apparent to those of ordinary skill in the art without departing from the scope and spirit of the disclosure. The embodiment was chosen and described in order to best explain the principles of one or more aspects set forth herein and the practical application, and to enable others of ordinary skill in the art to understand one or more aspects as described herein for various embodiments with various modifications as are suited to the particular use contemplated and in accordance with the following appended claims. Additional embodiments include any one of the embodiments described above and described in any and all exhibits and other materials submitted herewith, where one or more of its components, functionalities or structures is interchanged with, replaced by or augmented by one or more of the components, functionalities or structures of a different embodiment described above.

Accordingly, it is to be understood that the embodiments of the invention herein described are merely illustrative of the application of the principles of the invention. Other variations and modifications will be apparent to a person reading the description and as set forth in the following claims. Reference herein to details of the illustrated embodiments is not intended to limit the scope of the claims, which themselves recite those features regarded as essential to the invention.

Clauses of the Invention

1. A battery-free magnetic induction earplug comprising:

(a) a body including a sound channel, a coil cavity and an expansion cavity;

(b) a receiver in the coil cavity, the receiver including a coil of wire wound upon a bobbin of magnetic material;

(c) a speaker acoustically coupled to the sound channel, the speaker having a perforated back volume, a case, an acoustical output, and an electrical input, the case being at least partially made of magnetic material, the electrical input being coupled to the wire of the receiver, wherein the bobbin of the receiver is mounted in sufficiently close proximity to the case of the speaker and oriented such that flux lines passing through the speaker also pass through the coil of the receiver,

(d) the expansion cavity in acoustical communication with the back volume of the speaker.

2. The earplug of clause 1 in which the expansion cavity is located above the speaker and adjacent to the receiver.

3. The earplug of clause 2 in which the expansion cavity is contoured on the outer earplug surface above the speaker.

4. The earplug of clause 2 in which the expansion cavity is filled with acoustical absorption material.

5. The earplug of clause 2 in which the expansion cavity can be positioned at least partially between the tragus and antitragus when worn on a human ear.

6. The earplug of clause 2 in which the top surface of the expansion cavity generally slopes down from the receiver to the front of the earplug.

7. The earplug of clause 1 incorporating an equalization circuit connected to the receiver bobbin and speaker.

8. The earplug of clause 1 incorporating a front chamber adjacent to the speaker acoustical output.

9. The earplug of clause 1 incorporating a port that communicates between the expansion cavity and the acoustical output.

10. the earplug of clause 1 in which the speaker and receiver are oriented such that the axis of the receiver coil is generally perpendicular to a surface of the speaker case forming a generally right angle corner geometry where the receiver and speaker meet.

11. The earplug of clause 10 in which the expansion cavity is located, at least partially, in the right angle corner where the receiver and speaker meet.

**Claims**

1. A battery-free magnetic induction earplug comprising:

a housing defining:

a main cavity;
a sound output channel connected to the main cavity; and
an expansion cavity;

a receiver in the main cavity, the receiver including a bobbin and a coil of wire wound upon the bobbin; and
a speaker in the main cavity adjacent the receiver, the speaker acoustically coupled to the sound output channel, the speaker having a diaphragm and a volume upstream from the diaphragm, the speaker having a vent from the volume to the expansion cavity.

2. The earplug of claim 1, wherein the main cavity includes a speaker residual cavity between the speaker and the housing, and wherein the vent opens from the volume upstream of the diaphragm to the speaker residual cavity such that the speaker residual cavity acts as the expansion cavity.

3. The earplug of claim 1, wherein the main cavity includes a coil cavity between the coil of wire and the housing, and wherein the vent opens from the volume upstream of the diaphragm to the coil cavity via a sound path such that the coil cavity acts as the expansion cavity.

4. The earplug of claim 1, wherein the housing comprises:

   a first wall between the expansion cavity and the sound output channel, and between the expansion cavity and the main cavity; and
   a second wall between the expansion cavity and the main cavity,
   the first wall having a first opening therethrough between the expansion cavity and the main cavity,
   the vent aligned with and adjacent the first opening.

5. The earplug of claim 4, wherein the main cavity includes a coil cavity between the coil of wire and the housing, and wherein the second wall includes a second opening between the expansion cavity and the coil cavity.

6. The earplug of claim 4, wherein the first wall includes a second opening between the expansion cavity and the sound output channel.

7. The earplug of claim 1, wherein the housing comprises a first wall between the expansion cavity and the sound output channel.

8. Th earplug of claim 7, wherein the first wall includes an opening therethrough from the expansion cavity to the sound output channel.

9. The earplug of claim 1 wherein the expansion cavity is located adjacent the speaker and adjacent the receiver.

10. The earplug of claim 1 wherein the housing further comprises an outer wall defining at least a portion of the expansion cavity, the outer wall having a contoured outer surface.

11. The earplug of claim 10, wherein the contoured outer surface of the expansion cavity generally slopes from the receiver toward the sound output channel.

12. The earplug of claim 1 wherein the expansion cavity is filled with acoustical absorption material.

13. The earplug of claim 1, further comprising an equalization circuit connected to the coil of wire and the speaker.

14. The earplug of claim 1 wherein the speaker includes a speaker case housing the diaphragm, and wherein the speaker and the receiver are oriented such that an axis of the coil is approximately perpendicular to a surface of the speaker case forming a generally right angle corner where the receiver and speaker meet.

15. The earplug of claim 14 in which the expansion cavity is located, at least partially, in the right angle corner where the receiver and speaker meet.

16. The earplug of claim 1, wherein the housing further defines a speaker output chamber connecting the speaker to the sound output channel, the speaker output chamber having a radial dimension larger than a radial dimension of the sound output channel.

17. A battery-free magnetic induction earplug, the earplug comprising:
a housing defining a main cavity, a sound output channel connected to the main cavity, and an expansion cavity connected to the main cavity, the housing including a first wall between the expansion cavity and the sound output channel.

18. The battery-free magnetic induction earplug housing of claim 17, wherein the first wall includes an opening therethrough from the expansion cavity to the sound output channel.

19. The battery-free magnetic induction earplug housing of claim 17, wherein the first wall is between the expansion cavity and the main cavity, the first wall defining an opening between the expansion cavity and the main cavity.

20. The battery-free magnetic induction earplug housing of claim 17, wherein the housing further includes a second wall, the second wall being between the expansion cavity and the main cavity, the second wall extending approximately perpendicular to the first wall.

21. The battery-free magnetic induction earplug housing of claim 20, wherein the second wall defines an opening between the expansion cavity and the main cavity.

Fig. 1
Prior Art

33

1

35

31

Fig. 2
Prior Art

Fig. 3

33

57

W1/W2

40

35

31

Fig. 4

Fig. 5

Fig. 6

Fig. 7

Fig. 8

Fig. 9

Fig. 10

Europäisches
Patentamt

European
Patent Office

Office européen
des brevets

# EUROPEAN SEARCH REPORT

Application Number

EP 24 20 3379

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/322453 A1 (MATSUYAMA EIJI [JP] ET AL) 23 December 2010 (2010-12-23) | 1-21 | INV.<br>H04R1/10 |
| Y | * paragraphs [0002], [0024] - [0026]; figures 2,4 * | 1-21 | H04R1/28<br>A61F11/08 |
| Y | US 2021/352396 A1 (BLUNDELL MICHAEL PEARSON [US] ET AL) 11 November 2021 (2021-11-11) * paragraph [0075]; figures 2,5,6 * | 1-21 | |

TECHNICAL FIELDS SEARCHED (IPC)

A61F
H04R

The present search report has been drawn up for all claims

| Place of search | Date of completion of the search | Examiner |
|---|---|---|
| Munich | 13 February 2025 | Kunze, Holger |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**ANNEX TO THE EUROPEAN SEARCH REPORT**
**ON EUROPEAN PATENT APPLICATION NO.**

EP 24 20 3379

This annex lists the patent family members relating to the patent documents cited in the above-mentioned European search report.
The members are as contained in the European Patent Office EDP file on
The European Patent Office is in no way liable for these particulars which are merely given for the purpose of information.

13-02-2025

| Patent document cited in search report | | | Publication date | Patent family member(s) | | | Publication date |
|---|---|---|---|---|---|---|---|
| US 2010322453 | A1 | | 23-12-2010 | CN | 101606395 | A | 16-12-2009 |
| | | | | JP | 4921197 | B2 | 25-04-2012 |
| | | | | JP | 2008193449 | A | 21-08-2008 |
| | | | | KR | 20090110894 | A | 23-10-2009 |
| | | | | US | 2010322453 | A1 | 23-12-2010 |
| | | | | WO | 2008096529 | A1 | 14-08-2008 |
| US 2021352396 | A1 | | 11-11-2021 | AU | 2019335219 | A1 | 11-03-2021 |
| | | | | CA | 3109369 | A1 | 12-03-2020 |
| | | | | EP | 3847823 | A1 | 14-07-2021 |
| | | | | IL | 280740 | A | 29-04-2021 |
| | | | | US | 2021352396 | A1 | 11-11-2021 |
| | | | | WO | 2020051210 | A1 | 12-03-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 63586161 A
- US 10448143 B
- US 10357403 B2
- US 9083388 B
- US 9525930 B

### Non-patent literature cited in the description

- **YUAN et al.** Measurement of Pressure Discomfort Threshold in Auricular Concha for In-Ear Wearables Design. *Applied Ergonomics*, 2023